# EUROPEAN PATENT SPECIFICATION

(11) **EP 1 254 158 B1**
(45) Date of publication and mention of the grant of the patent: **19.10.2005**
(21) Application number: 01910214.4
(22) Date of filing: 02.02.2001
(51) Int. Cl.: C07K 1/113, C07K 14/375

(54) **METHOD OF STABILIZING A HYDROPHOBIN-CONTAINING SOLUTION AND A METHOD OF COATING A SURFACE WITH A HYDROPHOBIN**
VERFAHREN ZUR STABILISIERUNG EINER HYDROPHOBIN-ENTHALTENDEN LÖSUNG UND ZUR BESCHICHTUNG EINER OBERFLÄCHE MIT HYDROPHOBIN
PROCEDE PERMETTANT DE STABILISER UNE SOLUTION CONTENANT DE L'HYDROPHOBINE ET PROCEDE PERMETTANT DE REVETIR UNE SURFACE D'HYDROPHOBINE

(30) Priority: 04.02.2000 GB 0002660
(43) Date of publication of application: 06.11.2002
(73) Proprietor: Applied NanoSystems B.V., 9700 AC Groningen (NL)
(72) Inventor: DE VOCHT, Marcel, Leo, NL-3445 TE Woerden (NL); WÖSTEN, Herman, Abel, Bernard, NL-3705 SN Zeist (NL); WESSELS, Joseph, Gerard, Hubert, NL-9475 PG Midlaren (NL); ROBILLARD, George, Thomas, NL-9801 BB Zuidhorn (NL)
(74) Representative: Prins, Adrianus Willem
(86) International application number: PCT/NL2001/000082
(87) International publication number: WO 2001/057066

(56) References cited:
- US-A- 3 981 991
- US-A- 5 130 418
- US-A- 5 951 972
- DE VOCHT MARCEL L ET AL: "STRUCTURAL AND FUNCTIONAL ROLE OF THE DISULFIDE BRIDGES IN THE HYDROPHOBIN SC3." JOURNAL OF BIOLOGICAL CHEMISTRY, vol. 275, no. 37, 15 September 2000 (2000-09-15), pages 28428-28432, XP002175407 ISSN: 0021-9258
- PATENT ABSTRACTS OF JAPAN vol. 013, no. 575 (C-667), 19 December 1989 (1989-12-19) & JP 01 238600 A (NIPPON KOUTAI KENKYUSHO:KK), 22 September 1989 (1989-09-22)
- VOCHT DE M L ET AL: "STRUCTURAL CHARACTERIZATION OF THE HYDROPHOBIN SC3, AS A MONOMER AND AFTER SELF-ASSEMBLY AT HYDROPHOBIC/HYDROPHILIC INTERFACES" BIOPHYSICAL JOURNAL, NEW YORK, US, US, vol. 74, no. 4, April 1998 (1998-04), pages 2059-2068, XP000999824 ISSN: 0006-3495
- MARTIN G G ET AL: "ADSORPTION OF A FUNGAL HYDROPHOBIN ONTO SURFACES AS MEDIATED BY THEASSOCIATED POLYSACCHARIDE SCHIZOPHYLLAN" BIOPOLYMERS, NEW YORK, NY, US, vol. 49, no. 7, June 1999 (1999-06), pages 621-633, XP000999655 ISSN: 0006-3525
- MARTIN G G ET AL: "PURIFICATION OF A FUNGAL HYDROPHOBIN AND AN ASSOCIATED 17 KILODALTON PROTEIN" POLYMER PREPRINTS, AMERICAN CHEMICAL SOCIETY, US, vol. 39, no. 1, 1998, pages 347-348, XP001000355 ISSN: 0032-3934
- WESSELS J G H: "HYDROPHOBINS: PROTEINS THAT CHANGE THE NATURE OF THE FUNGAL SURFACE" ADVANCES IN MICROBIAL PHYSIOLOGY, ACADEMIC PRESS, LONDON, GB, vol. 38, no. 38, 1997, pages 1-45, XP000999791 ISSN: 0065-2911

## Description

The present invention relates to a method of stabilizing a hydrophobin-containing solution.

Hydrophobin-containing solutions must be handled carefully, as even modest shaking may result in the assembly of the hydrophobin resulting in aggregates which affect the ability to coat a surface as well as the uniform coating of a surface to be coated with said hydrophobin.

It is known that 100% trifluoroacetic acid (TFA) can be used to dissolve the aggregates. After removal of TFA by evaporation using a stream of gas the hydrophobin monomers obtained are taken up in water and used for coating. It has been found that this procedure can be repeated several times and that TFA has no adverse effects on hydrophobin. However, TFA is not a compound to be used for environmental and safety reasons as well as cost.

The object of the present invention is to reduce or eliminate the above disadvantages.

To this end a method according to the preamble is provided characterized in that the hydrophobin is subjected to a treatment with a disulphide bridge-cleaving agent to yield unfolded hydrophobin, said treatment involving the prevention of the formation of disulphide bridges from cleaved disulphide bridges, said treatment being chosen from the group consisting of i) a treatment with sulphite resulting in a modified hydrophobin carrying sulphite groups; ii) a treatment involving the prevention of the formation of disulphide bridges which comprises reacting hydrophobin reduced with a reducing agent with a sulfhydryl-protecting agent chosen to allow for removal of the sulfhydryl-protecting group formed yielding hydrophobin having removable sulfhydryl-protecting groups; and iii) a treatment involving the prevention of the formation of disulphide bridges which comprises exposing hydrophobin reduced with a reducing agent to an environment in which substantially no oxidizing agent is present.

It has been found that using the above method, it is easy to prevent a hydrophobin-containing solution from becoming turbid while, for example, transporting or handling the hydrophobin-containing solution.

Hydrophobins are a well-defined class of proteins (ref. 1) capable of self-assembly at a hydrophobic-hydrophilic interface, and having a conserved sequence
Xₙ-C-X₅₋₉-C-C-X₁₁₋₃₉-C-X₈₋₂₃-C-X₅₋₉-C-C-X₆₋₁₈-C-Xₘ
X, of course, represents any amino acid, and n and m, of course, independently represent an integer. In general, a hydrophobin has a length of up to 125 amino acids. The cysteine residues (C) in the conserved sequence are part of disulfide bridges. In the present invention, the term hydrophobin has a wider meaning to include functionally equivalent proteins, and encompasses a group of proteins comprising the sequence or parts thereof
Xₙ-C-X₁₋₅₀-C-X₀₋₅-C-X₁₋₁₀₀-C-X₁₋₁₀₀-C-X₁₋₅₀-C-X₀₋₅-C-X₁₋₅₀-C-Xₘ
still displaying the characteristic of self-assembly at a hydrophobic-hydrophilic interface resulting in a protein film. In accordance with the definition of the present invention, self-assembly can be detected by adsorbing the protein to Teflon and use Circular Dichroism to establish the presence of a secondary structure (in general α-helix) (ref. 2). The formation of a film can easily be established by incubating a Teflon sheet in the protein solution followed by at least three washes with water or buffer (ref. 3). The protein film can be visualised by any method, such as labeling with a fluorescent compound or by the use of fluorescent antibodies, as is well established in the art. m and n may have values ranging from 0 to 2000. Included in the definition are fusion-proteins of a hydrophobin and another protein.

The treatment with sulphite resulting in a modified hydrophobin carrying sulphite groups can be performed as described by Chan (ref. 6), results in a stabilized modified hydrophobin.

The use of a reducing agent as the disulphide bridge cleaving agent, resulting in the modified hydrophobin carrying free sulfhydryl-groups, is known in itself. Such sulfhydryl groups can be stabilized by one of several ways, for example using a sulfhydryl-protecting agent. Sulfhydryl-protecting agents, which are commercialy avialable, are agents capable of binding to the sulphur atom of a cystein residue, commonly by replacing the hydrogen atom of the sulfhydryl group. Accordingly, according to a preferred embodiment, the prevention of the formation of disulphide bridges comprises reacting the reduced hydrophobin with a sulfhydryl-protecting agent yielding hydrophobin having sulfhydryl-protecting groups.

Absence of an oxidizing agent, including atmospheric or dissolved oxygen, helps to prevent the formation of disulphide bridges.

De Vries, O.M.H. et al. (in Arch. Microbiol. 159, pp. 330 - 335 (1993)) investigated the state of cystein residues present in hydrophobin. By reducing or not reducing hydrophobin with DTT followed by carboxymethylation with iodol [2-³H]acetic acid, it was found that all cystein residues are involved in intramolecular disulphide bridges. This publication does not describe stabilization of a hydrophobin.

Preferably the sulfhydryl-protecting agent is a protecting agent resulting in an ionic protecting group.

While sulfhydryl-protecting agents in general and a sulfhydryl-protecting agent resulting in a bulky protecting group in particular are thought to be suitable for the purpose of protecting sulfhydryl groups resulting from the reduction of disulphide bridges, a sulfhydryl-protecting agent resulting in an ionic group (present after the sulfhydryl group is protected) is considered best.

According to a highly preferred embodiment, the sulfhydryl-protecting agent is chosen to allow for removal of the sulfhydryl-protecting groups to yield free sulfhydryl residues.

The removal allows the stabilized hydrophobin-containing solution to be used for coating a surface with previously stabilized hydrophobin, and may result in a coating which is more similar to a coating with untreated hydrophobin, with cystin residues being restored.

According to a preferred embodiment the reduction is performed in the presence of an agent chosen from the group consisting of a) a surfactant; b) a chaotropic agent, such as urea.

The use of such an agent, more in particular a protein unfolding-enhancing agent, facilitates the reduction of disulphide bridges present in hydrophobin.

Wessels, J.G.H. describes in Advances in Microbial Physiol. 38, pp. 1-45 possible applications of hydrophobins. Specific mention is made of the use of hydrophobins to enhance the biocompatibility of medical implants, including artificial bloodvessels and surgical instruments, and also biosensors. In accordance with the above, the present invention relates to a method for coating a surface with a hydrophobin, characterized in that a stabilized hydrophobin-containing solution according to the present invention is used, wherein the stabilized solution is contacted with a surface to be coated with the hydrophobin before the surface is contacted with an agent for the formation of disulphide bridges, and sulfhydryl-protecting residues, if present, are removed.

According to an alternative embodiment, the present invention relates to a method of coating a surface with a hydrophobin, characterized in that a stabilized hydrophobin-containing solution according to the present invention is used, wherein in the absence of a gaseous phase i) sulfhydryl-protecting groups, if present, are removed and ii) the reduced hydrophobin is contacted with an agent in the liquid phase before, during or after contacting the reduced hydrophobin with the surface to be coated.

Both these methods allow for the uniform coating of a surface without aggregates. Also, in both cases the agent is preferably an oxidizing agent.

Surprisingly it has been found that the presence of sulfhydryl-protecting groups does not impede the coating of a surface with a hydrophobin carrying said groups. The sulfhydryl-protecting groups may be removed at any time before, during or after the coated surface is contacted with the oxidizing agent.

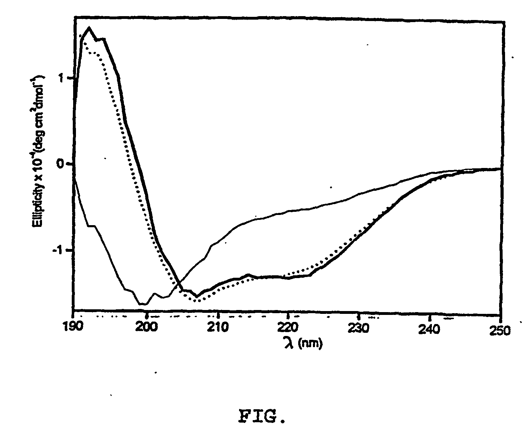

The invention will now be illustrated with reference to the following example and the only figure which shows a Circular Dichroism spectrum of a modified hydrophobin adsorbed to a Teflon surface.

### PREPARATIONS

### A) Purification of hydrophobin SC3

The hydrophobin SC3 was purified from the culture medium of strain 4-40 of *Schizophyllum commune* (CBS 340.81) as described (1, 4). Before use, the freeze-dried SC3 was disassembled with pure TFA and dried in a stream of nitrogen. The monomeric protein was then dissolved in the buffer specified under B), C) and D)

### B1) Carboxymethylation of SC3 with iodoacetic acid

Reduction of SC3 and carboxymethylation of the free cysteine residues were performed essentially as described by Hollecker (5). 1 mg of SC3 as obtained under A) was incubated for 30 minutes in 0.5 ml buffer containing 75 mM Tris/HCl pH 8.0, 5.4 M Guanidine Hydrochloride, 2.5 mM EDTA and 1 mM DTT at 37°C. This was followed by adding 50 µl of 0.2 M iodoacetic acid (IAA) in 75 mM Tris pH 8.0 and incubating the mixture for 15 minutes at room temperature. After reaction the sample was dialysed exhaustively against water and lyophilized, yielding IAA-SC3.

### B1) Sulfytolysis of SC3

Sulfytolysis results in reduction of disulfide bridges, with the concommitant formation of SO₃- groups, rendering the resulting protein derivative more soluble. The modification is a reversible modification. SC3 was sulfytolized essetially according Chan (ref. 6). In short, 2 mg SC3 was incubated overnight in 2 ml of a buffer (pH 8.4; 0.2 M sodium sulphite, 0.1 M Tris, 6 M guanidine hydrochloride, and 1 mM cystein) for 16 h at room temperature (RT). The reaction mixture was desalted using a Pharmacia PD-10 column. The reaction was checked using SDS-PAGE, which revealed a band at 28 kDa. After lyophilising, the resulting SO₃-SC3 was used in refolding experiments.

### C) Secondary structure measurements

The secondary structure of the carboxymethylated SC3 was studied with circular dichroism spectroscopy (CD). The CD-spectra were recorded over the wavelength region 190-250 nm on an Aviv 62A DS CD spectrometer (Aviv Associates, Lakewood, New Jersey, USA), using a 1-mm quartz cuvette. The sample compartment was continuously flushed with N₂ gas and the temperature was kept constant at 25°C. 10 scans were averaged, using a bandwidth of 1 nm, a stepwidth of 1 nm, and 1 sec averaging per point. The spectra were corrected using a reference solution without the protein. Typically a protein concentration of 10 µM in 20 mM phosphate pH 7.0 was used. To obtain spectra of the protein assembled on the water-air interface the solution was vigorously shaken for two minutes. For spectra of SC3 bound to a hydrophobic support, 130 nm unstabilized colloidal Teflon spheres (Dupont de Nemours, Geneva, Switzerland) in water were added to the solution, following a known procedure (2).

### D) Binding to Teflon

The coating of Teflon (Norton Fluorplast B.V., Raamsdonksveer, The Netherlands) by-SC3 and IAA-SC3 was assessed essentially as described by Wösten et al. (3). Thoroughly cleaned (ref. 3) Teflon sheets were incubated for 16 hours in 20 µg/ml ³⁵S-labelled hydrophobin in water, followed by three washes with water for 10 minutes each. The amount of adsorbed ³⁵S-labelled protein was determined by scintillation counting before and after hot SDS extraction (2%; pH 1,5) and subsequent washes with water.

### EXAMPLE 1

Solutions were prepared of 200 µg/ml SC3 and IAA-SC3, each in the buffer described under C) were shaken vigorously. Whereas a precipitate formed readily in case of SC3, the solution containing IAA-SC3 remained clear. This indicates that the solution containing modified hydrophobin is effectively stabilized.

### EXAMPLE 2

Upon addition of colloidal Teflon IAA-SC3 folded to the α-conformation, as observed with CD (thick solid line in the figure). SC3 adsorbed to colloidal Teflon also has the α-conformation (dotted line). Although CD-measurements showed that IAA-SC3 was unfolded in solution (thin line; even after shaking), the refolding of IAA-SC3 on Teflon shows the high propensity of stabilized hydrophobin to refold at such hydrophobic surfaces.

### EXAMPLE 3

SC3 binds very strongly to Teflon. Even heating for 10 minutes at 100°C in 2% SDS barely reduces the amount of hydrophobin adsorbed to a Teflon sheet. With IAA-SC3 the observed reduction in bound radioactivity was 16% versus 10% for SC3. This indicates a strong binding of modified SC3 under the test conditions.

With atomic force-microscopy, a typical rodlet pattern is observed with a hydrophobin such as SC3 dried on a flat mica surface. This same pattern was observed with IAA-SC3 (data not shown).

### EXAMPLE 4

Example 1 was repeated with SO₃-SC₃. The protein was soluble in water and did assemble or aggregate, even after shaking the solution. The CD spectrum was characteristic for unfolded protein (result not shown).

### EXAMPLE 5

Following the conditions of example 2, it was observed that SO₃-SC3 refolded at a Teflon surface, and CD showed the characteristic α-helical conformation (results not shown). The refolding is thought to be beneficial for the formation of native disulphide bridges (cystin).

### EXAMPLE 6

50% of radioactively labelled SO₃-SC3 bound to a Teflon surface remained bound after treatment with hot SDS at pH=7.0. At this pH, the sulphite groups of SO₃-SC3 are negatively charged. As a control, under the same conditions IAA-SC3 remained bound for 20% at this pH (84% remained bound at pH = 1.5). This difference in binding can be attributed to the reversible derivatization of the sulfhydryl groups in SC3 and subsequent formation of disulfide bridges. That is, these results can be interpreted that at least part of the bound SO₃-SC3 is refolded and some of the SO₃⁻-groups are removed, possibly by oxidation by oxygen present in the solution and resulting in the formation of disulphide bridges, resulting in a partial restoration of the original binding characteristics.

### REFERENCES

1. Wessels, J.G.H. (1997) in Adv. Microb. Physiol. 38, pp. 1-45.
2. De Vocht, M.L., et al. (1998) in Biophys. J. 74, pp. 2059-68.
3. Wösten, H.A.B., et al. (1994) in Embo. J. 13, pp. 5848-54.
4. Wösten, H.A.B., et al. (1993) in Plant Cell 5, pp. 1567-74.
5. Hollecker, M. (1989) in Protein Structure, ed. Creighton, T.E. (IRL Press, Oxford), pp. 145-53.
6. Chan W.W.C.,. (1968), Biochemistry, 7, pp. 4247-53.

## Claims

1. Method of stabilizing a hydrophobin-containing solution, **characterized in that** the hydrophobin is subjected to a treatment with a disulphide bridge-cleaving agent to yield unfolded hydrophobin, said treatment involving the prevention of the formation of disulphide bridges from cleaved disulphide bridges, said treatment being chosen from the group consisting of i) a treatment with sulphite resulting in a modified hydrophobin carrying sulphite groups; ii) a treatment involving the prevention of the formation of disulphide bridges which comprises reacting hydrophobin reduced with a reducing agent with a sulfhydryl-protecting agent chosen to allow for removal of the sulfhydryl-protecting group formed yielding hydrophobin having removable sulfhydryl-protecting groups; and iii) a treatment involving the prevention of the formation of disulphide bridges which comprises exposing hydrophobin reduced with a reducing agent to an environment in which substantially no oxidizing agent is present.

2. Method according to claim 1, **characterized in that** the sulfhydryl-protecting agent is a protecting agent resulting in an ionic protecting group.

3. Method according to claim 1 or 2, **characterized in that** the treatment is performed in the presence of an agent chosen from the group consisting of a) a surfactant; and b) a chaotropic agent.

4. Method for coating a surface with a hydrophobin, **characterized in that** a stabilized hydrophobin-containing solution according to any of the claims 1 to 3 is used, wherein the stabilized solution is contacted with a surface to be coated with the hydrophobin before the surface is contacted with an agent for the formation of disulphide bridges, and sulfhydryl-protecting residues, if present, are removed.

5. Method according to claim 4, **characterized in that** the agent is an oxydizing agent.

6. Method of coating a surface with a hydrophobin, **characterized in that** a stabilized hydrophobin-containing solution according to any of the claims 1 to 3 is used, wherein in the absence of a gaseous phase i) sulfhydryl-protecting groups, if present, are removed and ii) the reduced hydrophobin is contacted with an agent in the liquid phase before, during or after contacting the reduced hydrophobin with the surface to be coated.

7. Method according to claim 6, **characterized in that** the agent is an oxydizing agent.

## Patentansprüche

1. Verfahren zum Stabilisieren einer Hydrophobin-haltigen Lösung, **dadurch gekennzeichnet, dass** das Hydrophobin einer Behandlung mit einem Disulfidbrückenabspaltungsagens unterzogen wird, sodass entfaltetes Hydrophobin entsteht, wobei die Behandlung die Vermeidung der Bildung von Disulfidbrücken aus abgespaltenen Disulfidbrücken einschließt, wobei die Behandlung aus der Gruppe gewählt ist, die aus folgenden Komponenten besteht: 1) eine Behandlung mit Sulfit, die zu einem modifizierten Hydrophobin führt, das Sulfitgruppen trägt, II) eine die Vermeidung der Bildung von Disulfidbrücken einschließende Behandlung, die das Reagieren von mit einem Reduktionsmittel reduziertem Hydrophobin, mit einem Sulfhydryl-Schutzmittel umfasst, das gewählt wurde, um das Eliminieren der entstandenen Sulfhydryl-Schutzgruppe zu ermöglichen, sodass Hydrophobin mit eliminierbaren Sulfhydryl-Schutzgruppen entsteht; und III) eine die Vermeidung der Bildung von Disulfidbrücken einschließende Behandlung, in der mit einem Reduktionsmittel reduziertes Hydrophobin einer Umgebung ausgesetzt wird, in der im Wesentlichen kein Oxidationsmittel vorhanden ist.

2. Verfahren nach Anspruch 1, **dadurch gekennzeichnet, dass** das Sulfhydryl-Schutzmittel ein Schutzmittel ist, das zu einer lonenschutzgruppe führt.

3. Verfahren nach Anspruch 1 oder 2, **dadurch gekennzeichnet, dass** die Behandlung in Gegenwart eines Agens erfolgt, das aus der aus a) einem grenzflächenaktiven Stoff und b) einem chaotropen Agens bestehenden Gruppe gewählt ist.

4. Verfahren zum Beschichten einer Oberfläche mit einem Hydrophobin, **dadurch gekennzeichnet, dass** eine stabilisierte Hydrophobin-haltige Lösung nach einem der Ansprüche 1 bis 3 verwendet wird, wobei die stabilisierte Lösung mit einer mit dem Hydrophobin zu beschichtenden Oberfläche in Kontakt gebracht wird, bevor die Oberfläche mit einem Agens zur Bildung von Disulfidbrücken in Kontakt gebracht wird und, falls vorhanden, Sulfhydryl-Schutzgruppen eliminiert werden.

5. Verfahren nach Anspruch 4, **dadurch gekennzeichnet, dass** das Agens ein Oxidationsmittel ist.

6. Verfahren zum Beschichten einer Oberfläche mit einem Hydrophobin, **dadurch gekennzeichnet, dass** eine stabilisierte Hydrophobin-haltige Lösung nach einem der Ansprüche 1 bis 3 verwendet wird, wobei in Abwesenheit einer Gasphase 1) Sulfhydryl-Schutzgruppen, falls vorhanden, eliminiert werden und II) das reduzierte Hydrophobin mit einem Agens in der flüssigen Phase in Kontakt gebracht wird, bevor, während oder nachdem das reduzierte Hydrophobin mit der zu beschichteten Oberfläche in Kontakt gebracht wird.

7. Verfahren nach Anspruch 6, **dadurch gekennzeichnet, dass** das Agens ein Oxidationsmittel ist.

## Revendications

1. Procédé de stabilisation d'une solution contenant de l'hydrophobine, **caractérisé en ce que** l'hydrophobine est soumise à un traitement avec un agent de clivage des ponts disulfure pour produire de l'hydrophobine dépliée, ledit traitement impliquant la prévention de la formation des ponts disulfure à partir des ponts disulflure clivés, ledit traitement étant choisi parmi le groupe se composant de i) un traitement avec le sulfite ayant pour résultat une hydrophobine modifiée ayant des groupes de sulfite ; ii) un traitement impliquant la prévention de la formation des ponts disulfure qui comprend le fait de faire réagir l'hydrophobine réduite avec un agent réducteur avec un agent de protection à base de sulfhydryle choisi pour permettre la suppression du groupe de protection à base de sulfhydryle formé en produisant l'hydrophobine ayant des groupes de protection à base de sulfhydryle qui peuvent être supprimés ; et iii) un traitement impliquant la prévention de la formation des ponts disulfure qui comprend le fait d'exposer l'hydrophibine réduite avec un agent réducteur à un environnement dans lequel substantiellement aucun agent oxydant n'est présent.

2. Procédé selon la revendication 1, **caractérisé en ce que** l'agent de protection à base de sulfhydryle est un agent de protection ayant pour résultat un groupe de protection ionique.

3. Procédé selon la revendication 1 ou 2, **caractérisé en ce que** le traitement est exécuté en présence d'un agent choisi parmi le groupe se composant de a) un surfactant et b) un agent chaotropique.

4. Procédé pour revêtir une surface avec une hydrophobine, **caractérisé en ce qu'**une solution stabilisée qui contient de l'hydrophobine selon l'une quelconque des revendications 1 à 3 est utilisée, dans lequel la solution stabilisée est mise en contact avec une surface qui doit être revêtue avec l'hydrophobine avant que la surface ne soit mise en contact avec un agent pour la formation des ponts disulfure, et dans lequel les résidus de protection à base de sulfhydryle, s'ils sont présents, sont enlevés.

5. Procédé selon la revendication 4, **caractérisé en ce que** l'agent est un agent oxydant.

6. Procédé consistant à revêtir une surface avec une hydrophobine, **caractérisé en ce qu'**une solution stabilisée qui contient de l'hydrophobine selon l'une quelconque des revendications 1 à 3 est utilisée, dans lequel, en l'absence d'une phase gazeuse i) les groupes de protection à base de sulhydryle, s'ils sont présents, sont enlevés et ii) l'hydrophobine réduite est mise en contact avec un agent dans la phase de liquide avant, pendant ou après la mise en contact de l'hydrophobine réduite avec la surface qui doit être revêtue.

7. Procédé selon la revendication 6, **caractérisé en ce que** l'agent est un agent oxydant.
